(19) **Europäisches Patentamt European Patent Office Office européen des brevets**

(11) **EP 3 768 398 B1**

(12) **EUROPEAN PATENT SPECIFICATION**

(45) Date of publication and mention of the grant of the patent:
**27.12.2023 Bulletin 2023/52**

(21) Application number: **19773174.8**

(22) Date of filing: **17.07.2019**

(51) International Patent Classification (IPC):
*A63B 24/00* (2006.01)   *A63B 69/00* (2006.01)
*A63B 71/06* (2006.01)   *G01S 13/72* (2006.01)
*A61B 5/11* (2006.01)   *G06T 7/254* (2017.01)
*G01B 11/00* (2006.01)

(52) Cooperative Patent Classification (CPC):
**A61B 5/1122; A61B 5/1128; A63B 24/0021; A63B 69/002; A63B 71/0605; G01S 13/72; G01S 13/88; G06T 7/20;** A61B 2503/10; A63B 2024/0034; A63B 2220/30; A63B 2220/802; A63B 2220/89; A63B 2243/0025; G06T 2207/30224;   (Cont.)

(86) International application number:
**PCT/IB2019/056088**

(87) International publication number:
**WO 2020/016790 (23.01.2020 Gazette 2020/04)**

(54) **SYSTEM AND METHOD FOR OPTIMIZING A SPORTS BALL LAUNCH**

SYSTEM UND VERFAHREN ZUR OPTIMIERUNG DES ABWURFS EINES SPORTBALLS

SYSTÈME ET PROCÉDÉ POUR OPTIMISER UN LANCÉ DE BALLE DE SPORT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2018 US 201862699449 P**

(43) Date of publication of application:
**27.01.2021 Bulletin 2021/04**

(73) Proprietor: **Trackman A/S**
**2950 Vedbæk (DK)**

(72) Inventors:
• **HERMANSEN, Nicolaj Peter**
**2840 Holte (DK)**

• **TUXEN, Fredrick**
**2960 Rungsted Kyst (DK)**

(74) Representative: **Betten & Resch**
**Patent- und Rechtsanwälte PartGmbB**
**Maximiliansplatz 14**
**80333 München (DE)**

(56) References cited:
**WO-A1-2018/085894**    **KR-A- 20160 090 148**
**US-A1- 2008 312 010**    **US-A1- 2014 156 042**

(52) Cooperative Patent Classification (CPC): (Cont.)
G06T 2207/30241

## Description

### Technical Field

The present disclosure relates to a system and a method for optimizing a sports ball launch. In particular, the present disclosure relates to tracking a sports ball (e.g., a football, a soccer ball, etc.) in a kick situation so as to quantify the kicking technique of the kicking player, evaluate an expected performance of the kicking player in a given kick situation and to optimize a trajectory of the sport ball, in view of the kicking technique of the kicking player, to maximize the likelihood of scoring a goal.

### Background

To successfully launch a sports ball (e.g., to successfully execute a free kick (i.e. score a goal) in football (i.e. soccer) the player must launch the ball such that it is on target (directed at the goal), not blocked by any other players (teammates, opposing players, obstacles, etc.), and fast/hard enough so that an opponent (e.g., a goalkeeper) cannot reach the ball, taking into account the speed, abilities, and initial position of the opponent. In football (soccer), opposing players typically form a "wall" as the main defensive strategy for a direct free kick. A kicking player may typically avoid the wall by kicking above or around it by controlling the spin axis orientation and adding spin to the ball so that it remains on target. The kicking player's ability to control the spin axis and generate speed and spin is key to maximizing the likelihood of scoring a goal. However, kicking the ball at higher speeds may reduce the flight time, but the effect of the spin may not be sufficient to keep the ball on target. Thus, a balance must be achieved to strike the ball such that it avoids the wall, remains on target, and maintains enough speed to avoid the goalkeeper.

Official league statistics on direct free kick goal conversion rates in the top 5 European Leagues indicate that a few top performers consistently outperform all other designated free kick takers, with conversion rates above 25%, when observed over a 5-year period. The average team conversion rate is just 9%, indicating that significant improvement generally is possible for the majority of free kick takers - and potentially even the top performers can improve further.

WO 2018/085894 A1 discloses a method for determining whether a goal is achieved by a trajectory of a ball using a mobile computer device, comprising: capturing a sequence of video frames of the ball with a camera of the mobile computer device; detecting the ball in at least three of the video frames; computing a trajectory of the ball using the detections of the ball; detecting a goal image in at least one of the video frames; computing whether the trajectory of the ball achieves intersection or similar with a goal plane computed from the goal image according to a goal criterion.

## SUMMARY OF THE INVENTION

The invention is set out in the appended set of claims.

## SUMMARY OF THE DISCLOSURE

The present embodiments are directed to a method and a system for optimizing a sports ball launch. In particular, one of the present embodiments relates to a method which includes at a processor, identifying, from trajectory data for a kick, a plurality of kick parameters for a kicked ball and adjusting a value of a first one of the kick parameters to a first new value to calculate a first simulated trajectory in relation to a target area in combination with adjusting the value of the first kick parameter to a second new value to calculate a second simulated trajectory in relation to the target area and calculating a first blocked portion of the target area based on the first simulated trajectory and an obstacle between a launch position and the target area and a second blocked portion of the target area based on the second simulated trajectory and the obstacle. The method further includes determining an optimized value of the first kick parameter where the simulated trajectory of the kicked ball calculated with the optimized value enters an unblocked portion of the target area, the unblocked portion of the target area being the area remaining when the blocked portion of the target area is subtracted from the target area.

The present embodiments are further directed to a device comprising a database storing trajectory data for a kick and a processor identifying, from the trajectory data, a plurality of kick parameters for a kicked ball, the processor adjusting a value of a first one of the kick parameters to a first new value to calculate a first simulated trajectory in relation to a target area; the processor adjusting the value of the first kick parameter to a second new value to calculate a second simulated trajectory in relation to the target area; the processor calculating a first blocked portion of the target area based on the first simulated trajectory and an obstacle between a launch position and the target area and a second blocked portion of the target area based on the second simulated trajectory and the obstacle; and the processor determining an optimized value of the first kick parameter where the simulated trajectory of the kicked ball calculated with the optimized value enters an unblocked portion of the target area, the unblocked portion of the target area being the area remaining when the blocked portion of the target area is subtracted from the target area.

## Brief Description

Fig. 1 shows a tracking system for generating kick data and determining optimized kick parameters according to various exemplary embodiments of the present disclosure.

Fig. 2 shows the tracking system of Fig. 1 implemented on a football pitch.

Fig. 3 shows an exemplary flow chart for calculating a kick performance from a given set of kick data.

Fig. 4 shows the relationship between drag and lift coefficients relative to spin rate for a football moving at a given speed.

Fig. 5 shows a side view of the trajectories of kicks from a direct free kick series.

Fig. 6 shows the correlation between vertical launch angle and various launch parameters for a football.

Fig. 7 shows trajectory simulations for a set of free kicks with iterative launch parameter variations and a wall model.

Fig. 8 shows the principles of the goalkeeper model.

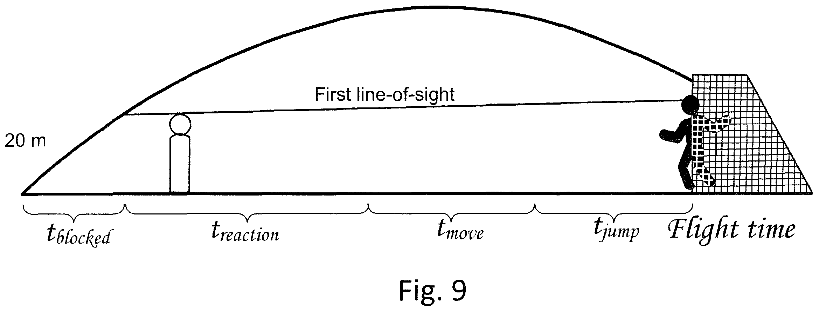

Fig. 9 shows a diagrammatic breakdown of the ball flight time into the components defining the save time for the goalkeeper.

Fig. 10 shows exemplary save times required for the goalkeeper to save the ball at any given goal crossing position.

Fig. 11 shows a goalkeeper area when the goalkeeper is positioned to fully cover one side of the goal.

Fig. 12 shows an exemplary wall positioned between the kicker and the goal.

Fig. 13 shows a top view of the wall of Fig. 12.

Fig. 14 shows trajectory simulations for a set of free kicks with iterative launch parameter variations and both a wall model and a goalkeeper model.

Fig. 15 shows an exemplary kick performance based on a kicker's kick data.

Fig. 16 shows the correlation between ball speed and various launch parameters for a football.

Fig. 17 shows an optimization process for maximizing the goal area for a kicker

Fig. 18 shows an exemplary decision evaluation for directing a free kick during a match.

## Detailed Description

The exemplary embodiments may be further understood with reference to the following description and the related appended drawings, wherein like elements are provided with the same reference numerals. The exemplary embodiments relate to a system and method for optimizing a sports ball launch. Some of the exemplary embodiments relate to analyzing kick data and optimizing a free kick situation to maximize a likelihood of a successfully scoring a goal. The parameters affecting the success of a free kick include the ability of the kicker, the position of the players on the pitch (particularly the position of the "wall" of the opposing team), the kicking location with respect to the opposing goal, the position and ability of the goalkeeper, the impact characteristics of the kicking boot with the ball, the aerodynamical properties of the ball, etc. Certain of these parameters may be modeled empirically by, e.g., tracking one or more kicks from a particular kicker and determining characteristics thereof to estimate the kicker's abilities or theoretically by, e.g., assuming a certain starting position and reaction time of a goalkeeper or an ideal positioning of a wall. Captured kick data may then be processed, taking into account pre-defined rules to, e.g., simulate a set of possible kicks achievable by the kicker and determine a trajectory that will maximize the chances of scoring for the given kick situation. For example, a kicking speed and/or spin on the ball at launch that maximizes the chance of converting the free kick may be presented to the kicker. In another embodiment, the kicker may have multiple shot types stored in the kick data, and the preferred shot type and optimized parameters for the given kick situation may be presented to the user. In still another embodiment, the optimizer may be used to present a theoretical optimized shot to a kicker to aid the kicker in mastering a certain shot type.

The term "wall" relates to a defensive strategy in a football game for protecting part of the goal from a direct free kick. The strategy includes positioning players a set distance from the free kick location, between the free kick location and the goal. The set distance may be determined by rule, e.g., a minimum of 10 yards from the ball. The "wall" may consist of only a single player, but typically includes a plurality of players joined together. The number of players used in a wall may vary based on the location of the ball with respect to the goal or other reasons. In practice, any type of blocking material may be used to simulate a wall for practice purposes. For example, the "wall" may be made of training tools, a rectangular structure, etc. sized to represent the area that opposing players would occupy in a football match.;

Although the exemplary embodiments will be described with respect to direct free kick situations in football (i.e. soccer) the exemplary embodiments are not limited thereto. For example, the exemplary embodiments may be adapted to other kicking situations such as, e.g., penalty shots, corner kicks and goalkeeper kicks, or kicks where the ball is not initially at rest. Additionally, certain of the exemplary embodiments may assume that the ball may not be curled around the wall (i.e., the ball may only be projected over the wall), however, the described principles may also be modified to encompass situations where the ball is curled around the wall. Further, the principles described herein may be adapted to other sports such as, e.g. American football, where kicker abilities may limit a team or kicker's strategy.

## System Diagram

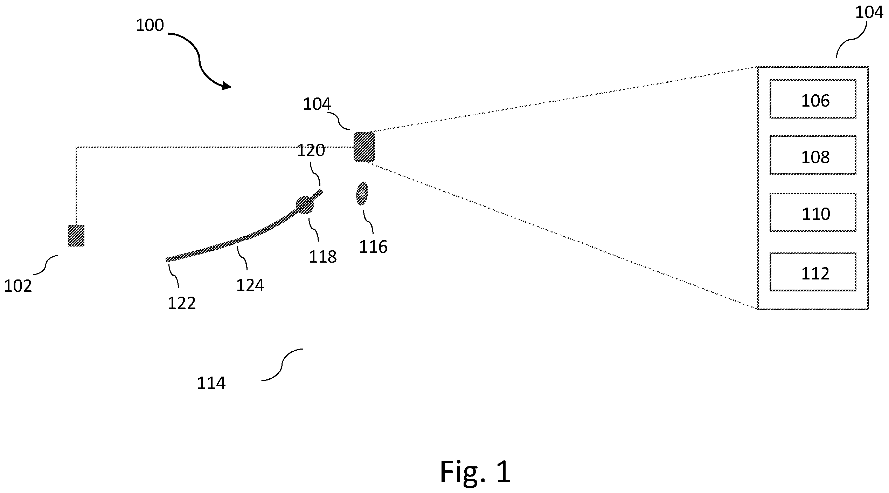

Fig. 1 shows a tracking system 100 for generating kick data and determining optimized kick parameters according to various exemplary embodiments of the present disclosure. The tracking system 100 includes a tracking device 102 for generating trajectory data of a launched ball 118. The tracking device 102 may be any sensor or plurality of sensors operable to identify and track a trajectory 124 of the ball 118 kicked by a kicker 116 from a launch location 120 to a landing location 122. For example, the tracking device 102 may comprise a camera, a radar, a LiDAR, an ultrasound, a micro-electro-mechanical sensor, or any number or combinations thereof. A preferred sensor for the tracking device 102 is a CW Doppler radar. The tracking device 102 may include some processing and data storage capabilities for generating and/or refining the raw data captured by the device 102, however, a large portion of the calculations described herein may be performed by a separate processing unit 106.

The tracking system 100 includes a computer 104 comprising a processor 106 for e.g. performing tracking and optimization calculations and a database 108 for e.g. storing tracking data and/or goalie data predetermined rules for processing the stored data. The computer 104 further includes a display 110 for e.g. presenting optimized kick parameters to a user and a user interface 112 for e.g. inputting a current kick situation (location, etc.). The various functions performed by the various elements of the computer 104 will be described in further detail throughout the present disclosure.

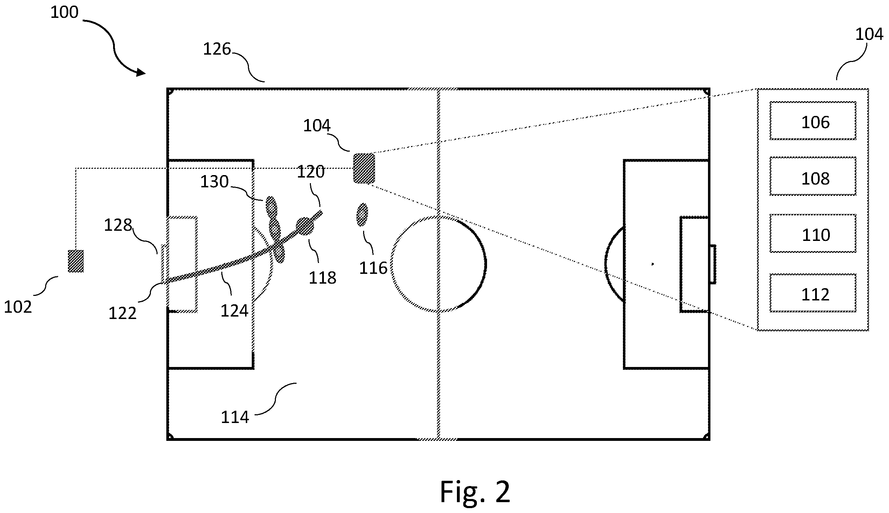

The tracking device 102 has a field of view (FOV) 114 covering an area that includes the launch location 120 and preferably the landing location 122, although the landing location will vary between kicks based on the trajectory of the struck ball 118. Fig. 2 shows the system 100 implemented at a football pitch 126 where the FOV 114 includes the launch location 120, a target location 128 (e.g., goal), and anticipated trajectory locations therebetween. However, the exemplary embodiments may be implemented at any location and are not limited to the football pitch 126. Similarly, the target location 128 may be any size and is not limited to the dimensions of a standard football goal. A wall 130 may be implemented between the target location 128 and the launch location 120.

## Kick Data

To generate kick data (i.e., kick trajectory data and launch parameters) the kicker 116 first strikes the ball 118 along the trajectory 124 from the launch location 120 to the landing location 122. If the system 100 is set up at the football pitch 126, the landing location 122 may be within the boundaries of the target location 128, or may be any other location. The tracking device 102 captures data on the entirety of the trajectory 124 or the entirety of that portion of the trajectory that remains within the field of play, however, portions of the trajectory 124 may be extrapolated when necessary, e.g., if a person or object interferes with the view of the ball 118 from the perspective of the tracking device 102. The measured trajectory data includes a minimum number of measurement times (t) with a corresponding position (x,y,z) of the ball 118 over the entire flight of the ball 118. Included in this data is preferably the position which corresponds to the impact time (t=0), i.e. the launch position 120, as the impact time and corresponding position would otherwise need to be determined through other means. The time steps in the trajectory sampling should allow for an accurate evaluation at any given position during the flight (the preferred time step is no bigger than 100 ms).

In addition to the trajectory measurement, the launch parameters are measured/determined for the struck ball 118 and are also considered part of the kick data. The launch parameters include an impact speed (i.e. speed at launch), a vertical launch angle, a horizontal launch direction, a spin rate at launch and a spin axis at launch. The launch parameters (other than spin rate) are derived from the trajectory data using standard and well-known methods. Spin rate (at launch) are estimated by applying some *a posteriori* knowledge about the ball aerodynamics, again using known approaches, which typically involve a fitting optimization process. For example, U.S. Pat. No. 8,845,442 describes methods for deriving a spin rate at launch of a spherical object, including footballs (soccer balls). By utilizing the same type of *a posteriori* knowledge about the ball aerodynamics, it is also possible to estimate the ball trajectory as a function of time even if only some of the launch parameters listed above were measured for a given kick, to be discussed in further detail below. This approach may degrade the accuracy of the results but will still produce a valuable result. However, in the preferred embodiment it is assumed that both the trajectory data as a function of time along, with the above-mentioned launch parameters, are measured and available.

The kick data, once determined, is stored in the database 108 and associated with the kicker 116. During the gathering of kick data, the kicker 116 should strike the ball 118 to the best of their ability and with a kicking motion that is representative of their typical kicking technique. The kicking technique of the kicker 116 drives the kicker's ability to generate speed, spin and orientation of the spin axis on the launched ball 118. Spin rate and orientation of the spin axis, in particular, may be difficult to adjust for the kicker 116, whereas parameters such as an impact location on the ball and a horizontal direction at launch, which are not correlated as closely with kicking technique are more easily adjustable. Speed may typically be adjusted downward from a maximum speed attainable by the kicker 116 but may not be easily adjusted upward.

The kick data may be based on a single kick or an averaging of a number of kicks. The kick(s) on which the kick data is based should reflect the kicker's preferred kicking motion and speed that the kicker 116 would use in typical gameplay. From this data, the processor 106 may simulate possible kick trajectories attainable by the kicker 116 when one or more of the launch parameters are varied. For example, the optimizer may adjust the vertical launch angle and horizontal launch direction, while keeping the spin and spin parameters the same. The various uses of the kick data will be described in further detail below.

Ball Aerodynamics

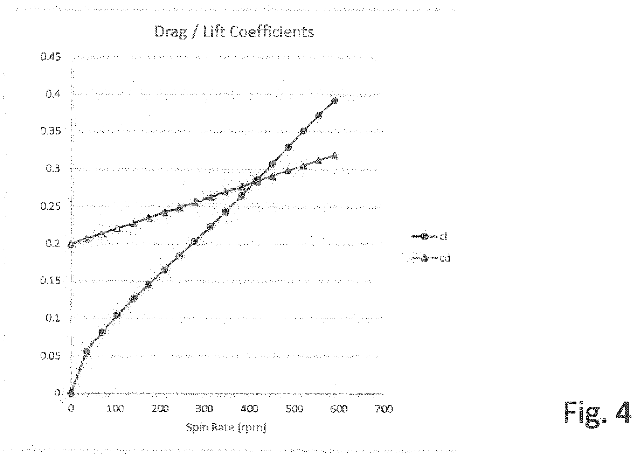

The kick data includes the aerodynamics of the ball 118 used during the gathering of the kick data. Factors affecting ball aerodynamics during flight include ball properties such as the ball dimensions, surface roughness, ball construction, weight distribution and panel shapes. Further, ball aerodynamics are affected by weather conditions such as air density, wind direction, wind speed and precipitation. The ball aerodynamics are preferably determined empirically through a series of sophisticated trajectory measurements performed under controlled conditions. In such a controlled investigation, parameters such as ball speed and spin rate are varied under some given weather conditions and stored in the database 108. For example, Fig. 4 shows an exemplary graph showing the lift coefficient ("cl") and the drag coefficient ("cd") for a known ball speed and a varying spin rate for a particular ball. The ball aerodynamics may be determined for a given mass-produced ball type and may be generally applicable to all balls of that type, unless the ball being simulated is, for example, significantly worn from use, improperly inflated, or has some other kind of imperfection. In general, both drag and lift forces will increase in magnitude when the spin rate is increased. Thus, increasing the spin rate generally increases the flight time for a given flight distance. These factors are accounted for when determining a flight time (between launch and crossing the plane of the goal), to be discussed in further detail below.

If ball aerodynamics are unavailable from an empirical analysis, then the aerodynamic consequences of adjusting a spin rate may not be accounted for in the parameter optimization. The actual drag and lift coefficients may be calculated for the measured kick data instead of using model coefficients, however, an accurate model projecting how the coefficients may vary for different spin conditions may not be generated from such limited data. Thus, in such a situation, there would be no need for the measurement device 102 to measure spin rate, as the consequence of spin rate would not be accounted for in the aerodynamic data. Such a degraded situation, while having utility, is not preferred. Thus, in the preferred embodiment, the ball type and its aerodynamic parameters (in view of the weather conditions) are known and stored on the database 108 prior to performing kick measurements/simulations. The weather conditions may come from a separate measurement device or a weather station that integrates with the system 100.

Flow Chart

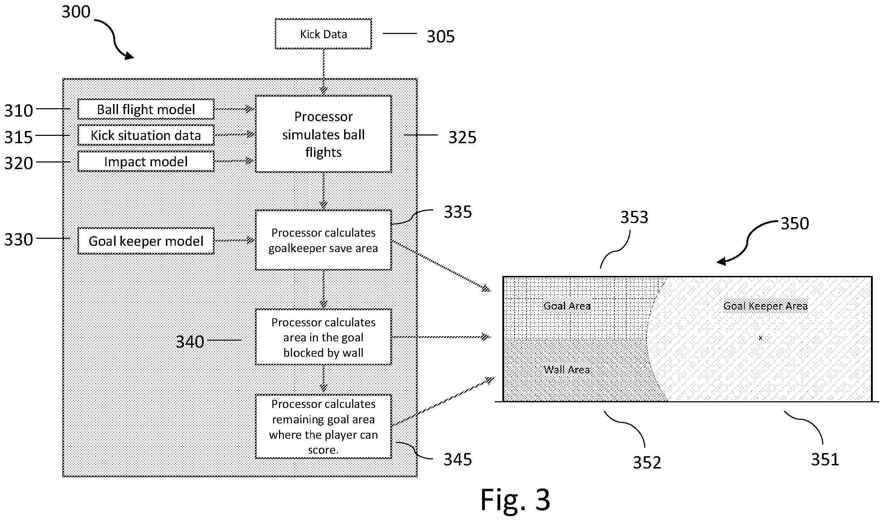

Fig. 3 shows an exemplary flow chart 300 for calculating a kick performance from a given set of kick data. Each of the data flows or processing steps depicted in the flow chart 300 will be briefly generalized in the following and described in greater detail throughout the present specification.

Kick data 305 is acquired by the system 100 for at least one kicker 116 based on at least one kick and stored in the database 108. As noted above, the kick data 305 includes trajectory data, launch parameters, and optional aerodynamic parameters for the ball 118 used for the at least one kick, and may encompass further information, to be discussed below. The kick data 305 may be based on a single kick or a plurality of kicks, from a single location or from a plurality of locations.

The ball flight model 310 refers to a simulated flight model for the ball 118 based on launch parameters and aerodynamic characteristics of the ball 118 and will be described in detail below.

Kick situation data 315 refers to an actual or theoretical kick situation on a football pitch 126 and includes a location of the ball 118 with respect to the goal 128, an arrangement of players on the pitch 126 (including the makeup of the wall) and an initial location of the goalkeeper. The derivation of kick situation data 315 will be described in detail below.

The impact model 320 refers to one of a plurality of options for modeling the impact between the kicker's boot and the ball 118. The impact model 320 may be a detailed characterization of a collision between two bodies, a model based on an analysis of a plurality of kicks where it is determined how the launch parameters correlate, or a simplified model where it is assumed that there is no correlation between certain of the launch parameters. The impact model 320 will be described in detail below.

In 325, the kick data 305, including trajectory data, launch parameters, and optional aerodynamic parameters for the ball 118 in varying weather conditions is processed in combination with the ball flight model 310, the kick situation data 315, and the impact model 320 to simulate a ball trajectory with one or more altered launch parameters. The result of step 325 is a grid of trajectory simulations where certain of the kicker's launch parameters (vertical launch angle and horizontal launch direction) are altered in small steps, to be described in further detail below.

The goalkeeper model 330 is a simulation of the goalkeeper's expected performance in blocking a kick, given some initial data regarding goalkeeper position and some parameters describing the goalkeeper's anticipated behavior, including e.g. his or her body dimensions, reaction time, and speed, to be described in further detail below.

In 335, an anticipated goalkeeper save area 351 is calculated based on the simulated ball flight times and the goalkeeper model 330. The goalkeeper save area 351 generally refers to an area in the plane of the goal 128 where the goalkeeper would be expected to save a kick, and thus should be avoided by the kicker 116 during the direct free kick.

In 340, a wall area 352 is calculated based on the simulated ball flights and a basic model of the wall. The wall area generally refers to an area in the plane of the goal 128 that is inaccessible to the kicker 116 based on the kicker's kicking technique and the blocking function of the wall.

In 345, a goal area 353 is calculated that is essentially the area remaining in goal once the goalkeeper area 351 and the wall area 352 have been subtracted. The goal area generally refers to an area in the plane of the goal 128 where the kicker 116 would be expected to score.

The result of the aforementioned calculations is a "Kick Performance" 350 for the kicker 116 in view of the kicker technique and the kick situation, to be described in further detail below.

Ball Flight Equations

Referring back to 310, a standard aerodynamic model is used to simulate the flight of the ball 118 using Newton's laws of motion in combination with drag and lift equations. To simulate the actual ball trajectory using the kick data and the aerodynamic data, including weather and ball data, the following equations may be applied in a ball flight model 310.

Newton's second law defines the acceleration $\hat{a}_{ball}$ of the ball throughout the flight:

$$\text{Equation (1):} \quad m_{ball}\hat{a}_{ball} = \sum F = F_{Gravity} + F_{Lift} + F_{Drag}$$

where $m_{ball}$ is the mass of the ball, $F_{Gravity}$ is the force from the gravity (defined by $m_{ball} * g$, where g is the gravitational acceleration), $F_{Lift}$ is the force induced by the spinning of the ball (lift force), and $F_{Drag}$ is the force from the air resistance (drag force). The magnitude of lift force is generally defined as:

$$\text{Equation (2):} \quad F_{Lift} = cl\frac{A}{2}\rho\, v^2$$

where cl is the lift coefficient, A is the cross-sectional area of the ball, $\rho$ is the air density and v is the speed of the ball relative to the wind. The magnitude of the drag force acting on the ball is generally defined as:

$$\text{Equation (3): } F_{Drag} \; = \; cd\frac{A}{2}\rho\, v^2$$

where cd is the drag coefficient. The drag force is opposite to the direction of the ball, and the lift force is directed in the direction of the cross product between the spin axis and the direction of the ball. Finally, gravity is directed towards the ground. A numerical implementation of the kinematic equations is then applied for calculating the trajectory:

$$\text{Equation (4): } \vec{x}_{n+1} = \vec{x}_n + \vec{v}_n \Delta t$$

$$\text{Equation (5): } \vec{v}_{n+1} = \vec{v}_n + \vec{a}_n \Delta t$$

where $\vec{x}_0$ is the kick location and $\vec{v}_0$ is the initial speed and direction of the ball (which is given by the launch speed, vertical launch angle and launch direction in the kick data). The ball acceleration is obtained from Equation (1) for any given point in time, with $F_{Drag}$ and $F_{Lift}$ coming from Equation (2) and Equation (3), using the empirically obtained aerodynamic data to calculate the drag coefficient cd and the lift coefficient cl.

Kick Situation

Referring back to 315, given the player kick data, a "kick performance" for a given situation may be evaluated by imposing a "kick situation" and simulating a kicker's performance given the kick situation. "Kick situation data" describes the kick situation and includes i) a location of the kick on the pitch (including a distance to the goal), ii) a position of the players on the pitch (including the wall), and iii) a position of the goalkeeper.

The kick situation data may be theoretical, where a fictional kick situation is generated, or actual, where the kick situation data is directly measured. For example, a player tracking system may be in use during a football match. This type of system is generally GPS-based or camera-based and is capable of determining the positions of all the players on the pitch on a continuous basis.

When analyzing a given direct free kick situation, it is preferable if the database 108 has kick data for the kicker from the actual free kick location. Thus, during the generation of the kick data, tracking kicks from multiple locations strengthens the kick data to better represent a variety of potential kick locations.

The kick situation data may be generalized for given situations. For example, referring back to Fig. 2, during the direct free kick situation the defensive wall 130 must be a minimum distance away from the kick location (e.g. 10 yards) and is typically formed at that distance. Additionally, the goalkeeper has time to take up an optimal position in the goal and the kicker may analyze the situation and adjust the strategy based on the disposition of players on the field. Further, the kicking team may position offensive players as an extension to the defensive wall 130 to block the view of the goalkeeper. This situation will be discussed further below with respect to the simulated goalkeeper performance. The generalized direct free kick situation disregards players on the pitch besides those forming the wall 130 and the goalkeeper.

Establishing an Impact Model

Referring back to 320, there are at least three options for simulating the launch of the ball 118 under adjusted launch/impact parameters based on the kick data. In the first option, a series of kicks are collected under varying launch conditions and the correlations between the different launch parameters are observed and modeled. In the second option, an impact model describing the collision between the kicker's foot and ball is used. This second approach requires the foot and ball collision to be measured and characterized in detail from a mechanics perspective as a collision between two bodies. In such a situation, the mechanical relationships between impact variables may be accurately accounted for. For example, if the impact location on the ball were changed as an isolated parameter as part of an optimization analysis (while everything else about the impact mechanics is kept the same), the resulting launch parameters would change in some correlated way described by the collision model. The collision may be modeled using e.g. finite element simulations or the like where the foot and the ball are described by some material parameters.

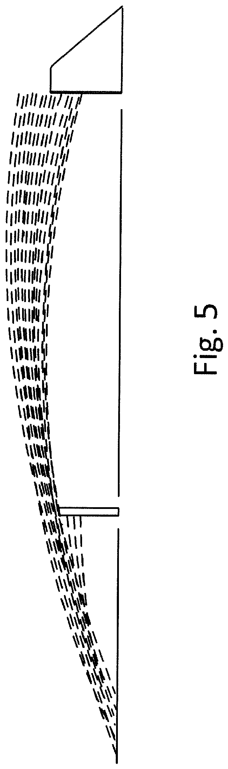

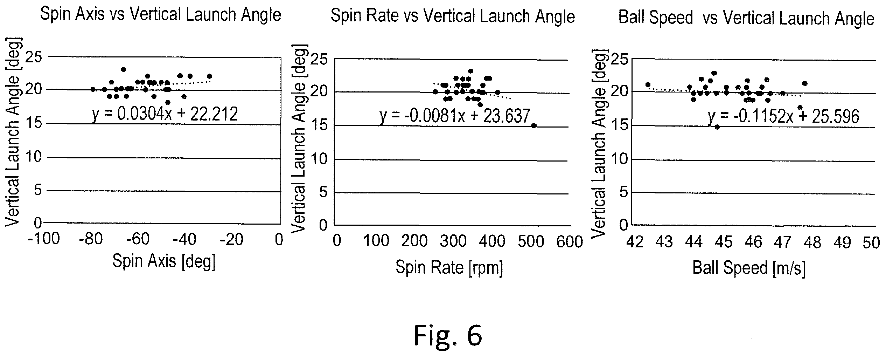

In the third and simplest option, the assumption is made that the launch parameters may be varied independently of one another. Specifically, it has been empirically validated by examining the data collected at several kick sessions with skilled (professional) free kick takers that the vertical launch angle of a launched ball may be varied independently of the other launch parameters (spin, spin axis orientation, ball speed). Fig. 5 shows a side view of the trajectories of kicks from a direct free kick series where the same player is attempting to replicate the same kick a number of times with varying launch angles. Each of the kicks was tracked and the kick data, including launch parameters, was determined. Figs. 6a-6c show plots of the vertical launch angle vs. each of the aforementioned launch parameters. As may be seen, there is little or no correlation between vertical launch angle and the launch parameters, indicating that simulating a potential change in the kicker's impact of the ball may be done simply by adjusting the vertical launch angle independently of the other launch parameters. This assumption has been found to apply for other free kick takers as well, although it is acknowledged that players have different kicking techniques and the observed independence across launch parameters may not apply generally across all players. Thus, a more advanced impact model, as discussed above, may provide a better simulation of flight data.

The horizontal launch direction may also be varied without affecting any of the other launch parameters; it is simply a matter of rotating the entire kick situation so that the exact same kick is executed but the ball is launched in a different direction relative to the goal. Adjusting the horizontal launch direction may affect the flight time to the crossing of the goal line, as well as the wall/goal crossing positions. However, it will not affect any of the launch parameters.

In sum, changing only the vertical launch angle is considered a representation of the kicker impacting the ball slightly later or earlier in their leg swing while essentially not changing the kicking technique otherwise, and also not kicking harder or softer (which may also be done without changing the kicking technique), while changing the horizontal launch direction is considered a representation of the kicker approaching the ball from a different direction while otherwise not changing the kicking technique. Using this approach, it is possible to examine all the possible kick options, i.e., the "kick repertoire," that a kicker would have given the kicker's current kicking technique.

Simulating Adjusted Ball Launches

Referring back to 325, given the assumption that vertical launch angle and horizontal launch direction may be varied independently of the other launch parameters, the processor 106 performs a simulation of ball trajectories with those parameters adjusted incrementally. Small step sizes (preferably not larger than .05deg per step) are used to adjust the vertical launch angle and the horizontal launch direction to generate a fine grid of trajectory simulations using the ball flight model 310 discussed above. The simulations are run such that, when the goal area 353 is reached by a simulated trajectory, the trajectory (and launch conditions) are stored as part of the player's kick repertoire. It is noted that, considering the ball has certain dimensions, to become a part of the kick repertoire the ball requires space to enter the goal (specifically half a diameter of space from the center of the ball to clear the goal posts completely, but slightly less when taking ricochets into the goal into account).

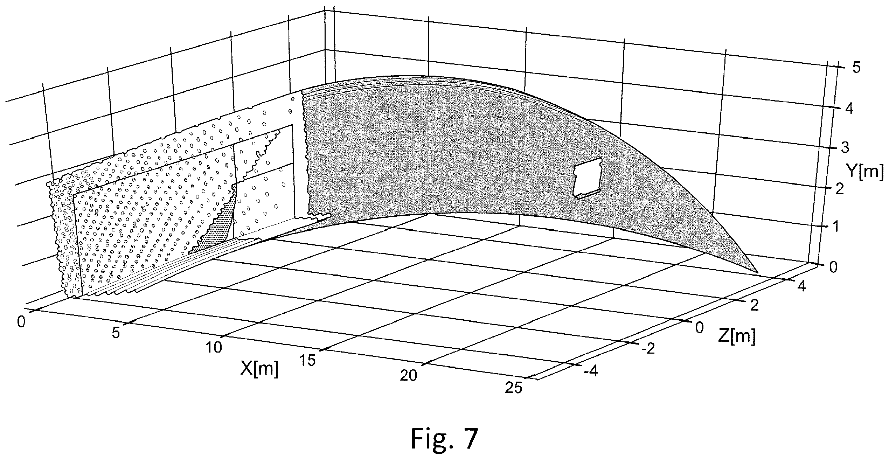

As mentioned above, in the preferred implementation the database 108 has the aerodynamic data of the ball stored thereon to make the ball flight simulation as accurate as possible. This empirically determined aerodynamic data may be adjusted or compensated as part of the simulation process to match the aerodynamic data derived from the actual measured ball flight, which may vary relative to the model due to a number of reasons (ball imperfections, simplifications / assumptions done when establishing the model, weather conditions that are not properly accounted for, difference in ball pressure etc.). This approach may be preferable if the aerodynamic data is not well-specified for the ball type being used, or if there is a large ball-to-ball variation that needs to be accounted for. The result of such a compensation is then stored in the database 108, as mentioned above. The principle of the ball launch variation (now reduced to a vertical launch angle and horizontal launch direction variation) and the corresponding simulations are depicted in Fig. 7, where a goal crossing area covering slightly more than the actual goal dimensions is covered by the simulation steps evaluated. An ultra-fine grid can in principle be evaluated, but to increase processing speed, interpolation between results can be applied instead.

Goalkeeper Model

Referring back to 330, each of the simulated kicks has an associated amount of time that it takes for the kick to cross the plane of the goal 128, i.e., a "flight time." Relatedly, for each goal-crossing position in the plane of the goal, the goalkeeper has a "save time," i.e., the amount of time that it takes for the goalkeeper to react, move and block a ball crossing the goal plane, given an initial goalkeeper position. If, for a simulated kick, the save time does not allow the goalie to reach the point at which the ball will cross the goal plane before the time at which the will cross the goal plane (as determined based on the flight time) then the simulated direct free kick will be successful.

To evaluate which of the simulated kick trajectories the goalkeeper would be able to save, an initial position of the goalkeeper is established at time t=0, i.e., the time the ball is kicked. A generalized approach is to assume that the goalkeeper will position him or herself in an optimal position, based on some criteria, e.g., the position where the "save area" (goalkeeper area 351) to be discussed in further detail below, is maximized, or the center of the goal, or some other criteria. However, another "non-ideal" position may be used for the calculation. For example, from a historical perspective, the goalkeeper typically takes a position in the goal from which he or she is expected to cover the area nearest to the far post, i.e., the goal post that is furthest from the kick location. Unless the goalkeeper has detailed knowledge of the kick repertoire of the kicker, it is unlikely that the goalkeeper will choose a most statistically optimized location. Thus, many different assumptions may be made about the initial goalkeeper position, depending on the situation to be simulated and the preferences of the user. However, using an optimized goalkeeper position, as determined in the manner described below, will generally provide a baseline worst-case scenario (i.e., minimize the potential goal-scoring area) from the kicker's perspective.

To simulate a goalkeeper's expected performance, with respect to defending against a shot on goal, a goalkeeper model 330 has been implemented and stored on the database 108. The goalkeeper model 330 simulates what could be expected from the goalkeeper given the flight times of the different kicks in the kicker's kick repertoire. The goalkeeper model may reflect an average performance, a less-skilled performance, or an elite performance. It may be tailored toward different playing levels, age groups, genders, etc. The model presented herein is a basic model but could easily be developed to include more complex details about the goalkeeper behavior.

The goalkeeper model presented here utilizes parameters based on video analysis of several direct free kicks taken at the highest level (top 5 leagues in Europe). There seem to be no previous studies performed that attempt to model the performance of the goalkeeper relative to the save time available. There have, however, been many studies done on the statistical performance of the goalkeeper in a penalty kick situation, where the goalkeeper does not have time to first analyze the ball flight and then react (at close range, this can also be the situation for direct free kicks). In these situations, the goalkeeper needs to act immediately upon impact, or, as in most cases, slightly before impact, but not so early that the player has time to adjust and kick the ball in a direction opposite to the goalkeeper's dive. In a 2006 study by Ken Bray and David Kerwin (Kerwin D.G., Bray K. (2006) Measuring and Modelling the Goalkeeper's Diving Envelope in a Penalty Kick. In: Moritz E.F., Haake S. (eds) The Engineering of Sport 6. Springer, New York, NY), the authors studied the goalkeeper's save area in a penalty kick situation at an elite level for men (where the goalkeeper has almost no time to move prior to jumping) in an attempt to identify, based on the goalkeeper's jumping ability, an "unsavable zone" in the goal which should then be the area for which penalty kickers should aim. The area that the goalkeeper was expected to cover based on this study was denoted the "diving envelope" of the goalkeeper, and this diving envelope principle is utilized in the model presented below, to define a maximum distance the goalkeeper can be expected to jump from a given position.

A basic model for men at the highest professional level contains the following variables, where the value stated next to each variable name corresponds to the average value determined from the video analysis discussed above:

*Arm span radius:* 0.9 m
*Leg span radius:* 1.2 m
*Leg height:* 0.9 m
*Shoulder height:* 1.5 m
*Reaction time:* 350 ms
*Jumping speed:* 5 m/s
*Moving speed:* 3.5 m/s
*Eye to shoulder distance:* 0.2 m
*Diving envelope radius:* 2.85 m
*Time before ball is visible:* This figure is trajectory dependent and needs to be determined using a given wall height combined with the actual trajectory being evaluated, to be described below.

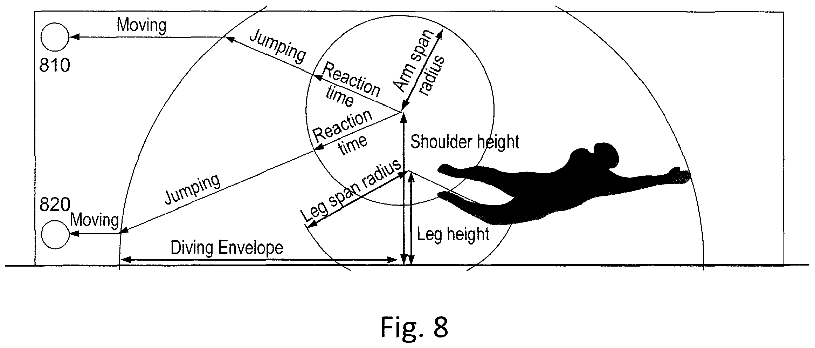

Fig. 8 shows the principles of the goalkeeper model.

Referring back to 335, the goalkeeper model evaluates how much time it would take the goalkeeper to save a ball at its goal crossing position from a given location of the goalkeeper. It is assumed that the arm span coverage is a perfect circle that has its center between the shoulders of the goalkeeper. The same principle is applied with the legs, although there is some upper angle as to how high the legs can be lifted within what is considered the "reaction time." In the model this upper angle is set to 50 degrees to each side measured from when the legs are in a vertical position. The leg span radius is however relevant only if the ball is kicked directly at the goalkeeper. Otherwise, the goalkeeper would save the ball using his/her hands. Thus, for direct free kicks, it is generally not important to include the leg span coverage.

To save the ball at a given goal crossing position (e.g. 810 or 820 in Fig. 8), the goalkeeper would first need to observe the ball within the time period referred to as the reaction time (i.e., the goalkeeper cannot start observing the ball until there is a line-of-sight to the ball). Next, the goalkeeper would need to move laterally the distance denoted *"Moving"* in Fig. 8. with his/her *"moving speed"* until the ball comes within the area of the diving envelope, where the goalkeeper would then need to move the distance denoted *"Jumping"* in Fig. 8 with his or her *"jumping speed"* until the goal crossing position of the ball is reached. The movement of the goalkeeper is modeled to happen from the position of the shoulder height as shown in Fig. 8, which is essentially the center of the circle with the "arm span radius." If the ball is within the indicated area of the arm span radius or the "leg span radius," the goalkeeper is assumed to save the ball. Formally, the following Equation (6) is how the time required for the goalkeeper to save the ball 810 or 820 in Fig. 8 would be calculated, assuming that the goalkeeper position and the goal crossing positions are both known:

$$\text{Equation (6): } t_{save} = t_{blocked} + t_{reaction} + t_{moving} + t_{jumping},$$

where $t_{blocked}$ is the time until the ball is visible for the goalkeeper (in a direct free kick situation, that would be the time it takes for the ball to clear the wall, in the sense that there is a line-of-sight from the goalkeeper to the ball). It is important to note that even though the goalkeeper would not typically setup a wall that would block his/her own view, the opposing team could benefit from setting up additional players to block the view. This situation would preferably be accounted for, as that generally increases the likelihood of scoring. The variable $t_{reaction}$ is the time period between the goalkeeper's observing the ball and determining its direction to estimate its goal crossing position. The variable $t_{moving} = \frac{d_{moving}}{v_{moving}}$ is the time it takes the goalkeeper to laterally move the distance $d_{moving}$ when moving with the speed $v_{moving}$. The goalkeeper is modeled as moving until the expected goal crossing position of the ball is just at the edge of the area that he can reach by jumping (as defined by the diving envelope). Finally, $t_{jumping} = \frac{d_{jumping}}{v_{jumping}}$ is the time it takes the goalkeeper to jump the distance $d_{jumping}$ with the jumping speed $v_{jumping}$. The goalkeeper generally jumps faster than he or she moves (as shown in the exemplary variables "jumping speed" and "moving speed" above), so it is beneficial to jump as soon as possible. Here, the moving and jumping speeds are assumed to be constant. The moving and jumping speeds may not be constant in reality, but as mentioned above the model may be made more complex if necessary. The breakdown of the ball flight time into the components defining the required save time, $t_{save}$, for the goalkeeper is shown in Fig .9.

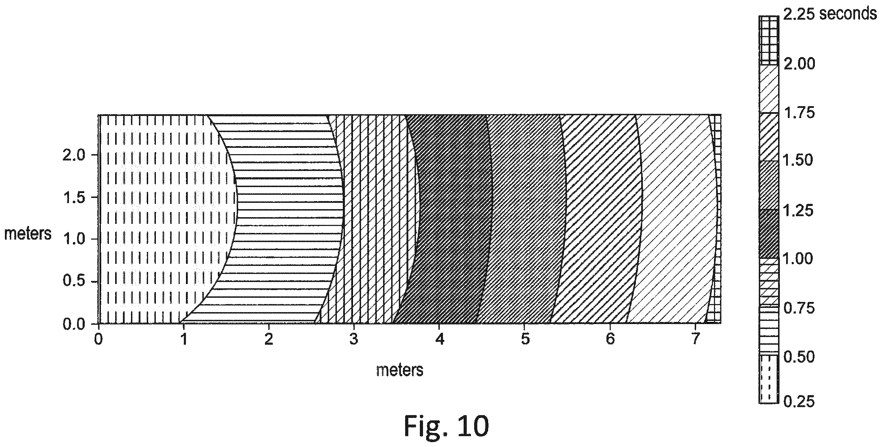

Evaluating the save time for a given goal crossing position relative to the goalkeeper may be calculated using the basic goalkeeper model. This evaluation leads to a matrix which is shown graphically by the contour plot in Fig. 10, which shows exemplary save times required for the goalkeeper to save the ball at any given goal crossing position. For example, if the goalkeeper is positioned at one goal post and tries to save a ball crossing at the other goal post, the goalkeeper would need to move the full distance of the goal width. The save time corresponding to that distance would be the largest save time shown in Fig. 10. Assuming that the goalkeeper has the same save time no matter which side of the goal is to be covered, then the save time to any goal crossing position in the goal can be determined from the matrix depicted in Fig. 10.

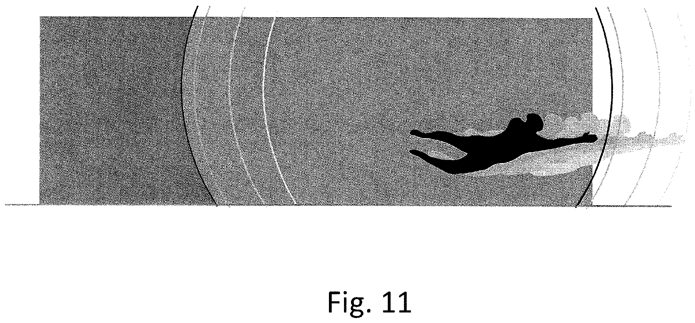

Assuming the optimal goalkeeper position is determined such that the far post is fully covered the goalkeeper area 351 may be similar to that shown in Fig. 11. Using the flight time of each of the ball flight trajectories stored in the kick repertoire, as depicted in Fig. 7, together with the save time for any given goal crossing position (while still taking into account that each trajectory has a period of time where the goalkeeper does not have a line-of-sight to the ball), the goalkeeper area may be evaluated for any given position of the goalkeeper in the goal. A wall height is also applied to determine the first line-of-sight, to be discussed in further detail below. Typically, such a height is between 1.8 - 2.4 m for men, assuming that the players in the wall are jumping. By "moving" the goalkeeper further and further away from the goal post the furthest away from the kick location, this evaluation may be repeated iteratively. The ideal goalkeeper position would then be the position where the goalkeeper allows the fewest possible ball trajectories to enter the goal, while also not allowing balls to enter the goal near the far post. For a given goal crossing position and goalkeeper position, the requirement would simply be that the following should be satisfied:

$$t_{save} = t_{blocked} + t_{reaction} + t_{moving} + t_{jumping} \leq t_{ball\,flight}$$

in order for the goalkeeper to save the ball. The result would then be the ideal position of the goalkeeper.

In addition to evaluating the kick performance, the goalkeeper model can also be used to evaluate the performance of the goalkeeper in relation to placement in the goal, the reaction time, the speed of the movements etc. A typical undesired action taken by the goalkeeper in a save situation is that he/she takes a step in the wrong direction relative to where the ball will eventually be crossing the goal line. Such an action takes precious time from the goalkeeper's saving ability and potentially will not allow him/her to save the ball or handle the ball well. That's one of the key reasons why opponents try to block the view of the goalkeeper or even try to make the goalkeeper make such an undesired movement. This situation could also be modeled or evaluated as part of a goalkeeper performance evaluation. Finally, the ball flight type referred to as a knuckleball in baseball, when the ball has some erratic movement during the flight is one situation also in soccer, where the ball can suddenly change its flight direction, which can have the effect that the goalkeeper needs to change direction, in which case critical time is lost for the goalkeeper, leaving less time to move and save the ball. This situation is special and generally not well described or understood in literature and would require some more advanced aerodynamic studies, before that could be directly evaluated. However, the principle of the invention does not exclude this ball flight behavior in any way.

Wall area

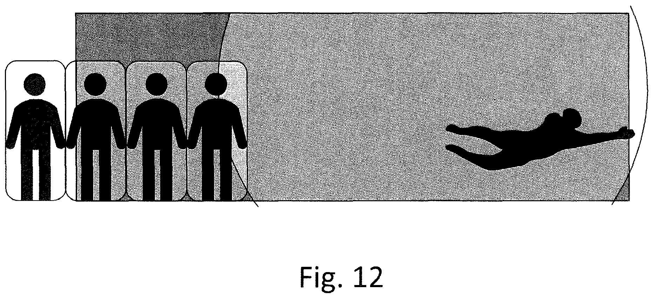

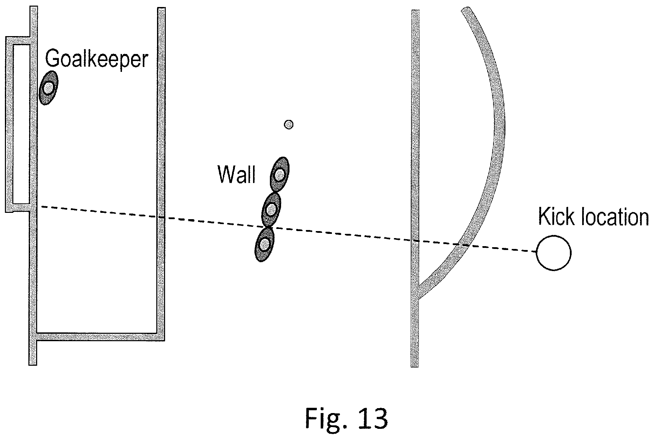

Referring back to 340, the calculation of the wall placement is less complicated. There is generally no need to position any players in the wall that will block the area already covered by the goalkeeper. By rule in professional football leagues, the wall must be positioned at least 10 yards away from the kick location. However, this number may vary depending on the context. By evaluating the position of each of the ball trajectories from the kick repertoire of the kicker, the first player in the wall may be placed so that no ball is able to pass the side of the wall on a straight path and enter the goal past the near goal post (at least not for the given trajectory shape, which is the premise of the *kick performance* evaluation). This principle is shown in Fig. 12. Additional players may be added to the wall until the portion of the width of the goal not covered by the goalkeeper is fully covered. A player width is assumed, which is generally 0.6m for males. A common rule of thumb in soccer is to position the first person in the wall so that he or she is covering an area that is actually outside of the goal relative to a direct line between the nearest goal post and the kick location. This principle is illustrated in Fig. 13 by the dashed line, where one player is positioned so he or she is blocking the outside of the goal, at least when considering a kick that would travel along a straight line.

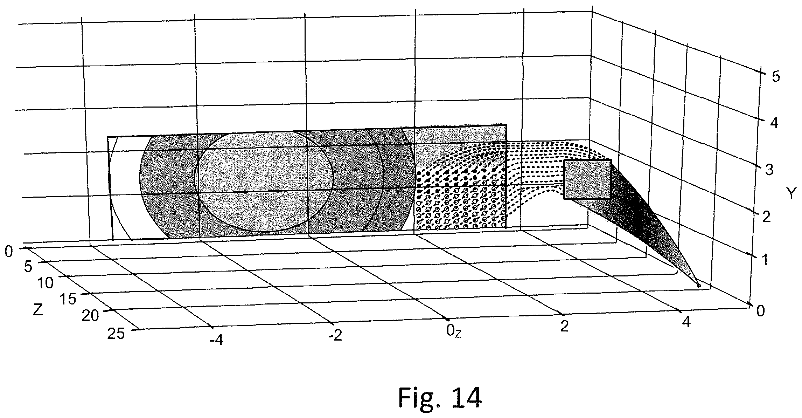

The wall area 352 is now calculated from the trajectories in the kick repertoire, which would have crossed the goal line had they not been blocked by the wall (assuming again some wall height, in this case a height of 2.2 m is applied). This principle is shown in Fig. 14., where the goalkeeper area 351 is also highlighted.

Goal area

Referring back to 345, the goal area 353 is now the remaining part of the goal, which is the area where the trajectories from the kick repertoire would not be blocked by either the goalkeeper or by the players standing in the wall. In general, one could argue that the bigger the goal area 353, the higher the likelihood of scoring, which may generally be the case, although some kick types that lead to a bigger goal area *353,* may also be more difficult to perform, reducing the reproducibility (or consistency), which is also a parameter. This is the case for the top spin kick, which generally increases the goal area 353 relative to other kick types, but compared to a side spin kick, the consistency is typically reduced (it is noted that no standard definitions exists as to what is considered a side spin, top spin, back spin or knuckleball kick, however, generally the behavior of the flight is defined by the combination of launch parameters, which may generally describe all possible kick types).

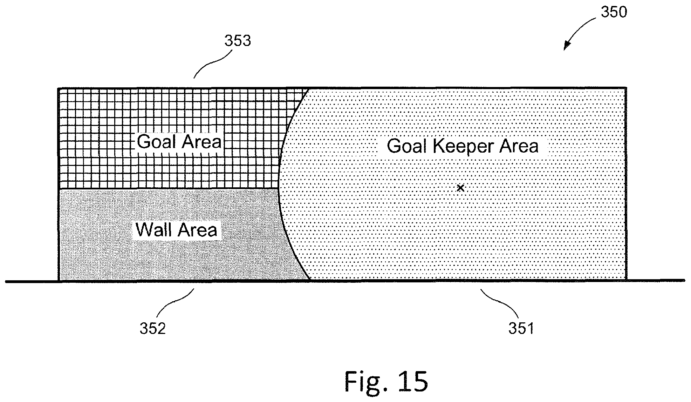

Referring back to 350, the kick performance 350 consists of the three areas: goalkeeper area 351, wall area 352 and goal area 353, as shown in Fig. 15. Once calculated by the processor 106, the kick performance 350 is shown on the display 110.

Kick Optimization

Once the kick performance has been fully analyzed for the kicker, in view of the actual or theoretical kick situation, the kick may be optimized. Up to this point the computer 104 has analyzed the current situation for the available kick data with incremental variances in vertical launch angle and horizontal launch direction, i.e., easily changeable variables, from the kicker's perspective. Given this information, the "kick optimization" process optimizes one or more of the other kick impact parameters to maximize the size of the goal area for the given kick data.

The optimization generally has two objectives, which may be applied independently or in combination: optimizing the kicking speed and/or the kicking technique so that the goal area is maximized. Changing the kicking speed is generally simple, from the kicker perspective, and may be considered a short term or immediate change. There is, of course, an upper limit as to how hard a player can kick the ball given his or her kicking technique, but up to a certain kicking distance most players are capable of adjusting their kicks to kick the ball at the optimized speed.

Changing the kicking technique, however, is more difficult. A change to a player's kicking technique may involve adjustments to the player's leg swing, run-up to the ball, positioning of the supporting leg, impact location on the player's foot, etc. Adjustments to the kicking technique are typically done to increase the amount of top spin by adjusting the orientation of the spin axis. There seems to be a limitation as to how much a player is able to change this technical ability. Thus, optimizing the kicking technique may require practice for the player, and may be considered a long term change, or a player development issue.

There are at least two options for simulating adjustments to the player's kicking speed. In the first option, the impact model discussed above with respect to determining correlations between launch parameters is used to determine and compensate for any dependencies between launch parameters. However, when the impact model is not available, the second option may be implemented, which assumes there is no correlation between the launch parameters.

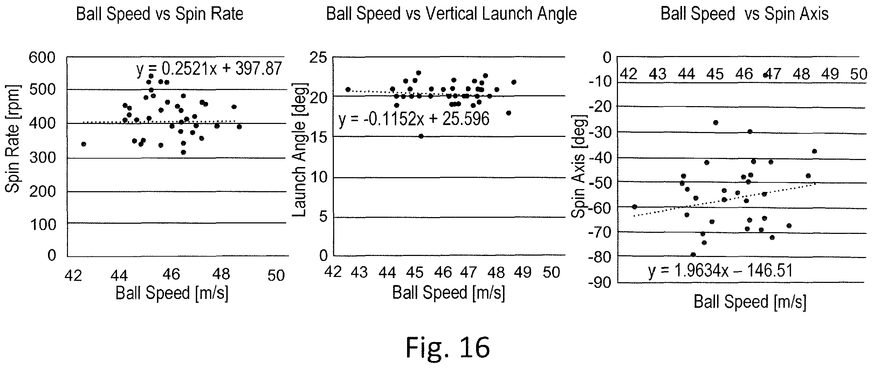

The same kick session data that was used above to ascertain correlations between a vertical launch angle and the remaining launch parameters, as shown in Fig. 6, may also be used to ascertain correlations between launch speed and the remaining launch parameters. Figs. 16a-c show plots of the ball speed vs. the parameters of spin rate, vertical launch angle, and spin axis. Similar to the vertical launch angle analysis, it may be seen that ball speed has little to no effect on spin rate or vertical launch angle, whereas there is a minor correlation found with respect to spin axis. However, for purposes of the present optimization, it is valid to neglect the cross-parameter dependency between ball speed and spin axis so long as the change in ball speed is relatively small. For example, a change in ball speed of less than 5 m/s is generally sufficient to reach the optimum speed.

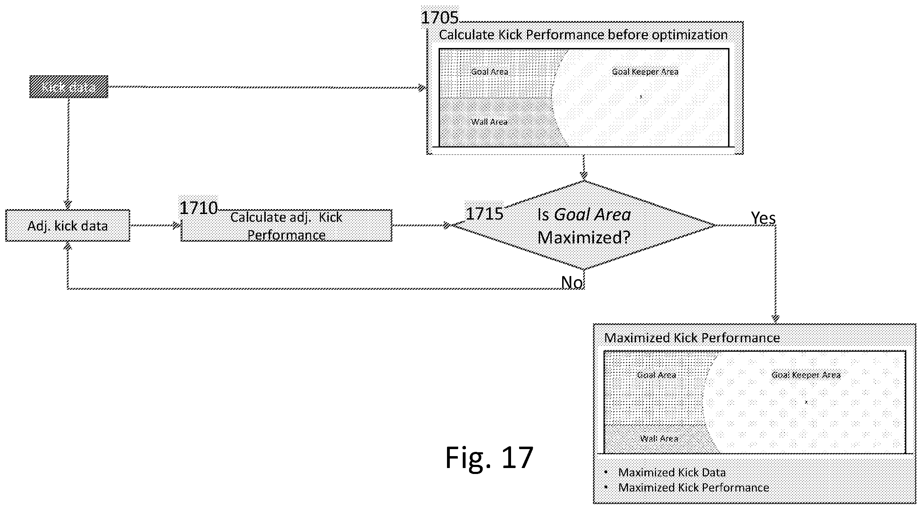

Fig. 17 shows an optimization process 1700 for maximizing the goal area for a kicker. In the present example, the optimized launch parameter is ball speed, however, the process may similarly be applied to other launch parameters. In 1705, the kick data for the kicker is processed to calculate a kick performance in accordance with the flowchart of Fig. 3.

In 1710, the same kick data is processed with adjusted launch parameters. The parameters are adjusted on an iterative basis with small steps to evaluate a corresponding change in the kick performance. In 1715, the adjusted kick performances are evaluated relative to one another to determine if a maximum goal area has been found.

Once the maximum goal area has been identified the corresponding kick data and kick performance are output as the optimized result. The results may be output to the display 110, or any other suitable output.

Match Situation

The optimization process described above may be used in an actual match situation to guide the selection of a kicker to take a free kick and the selected kicker's strategy for executing the free kick. The exemplary embodiments have been described heretofore in the context of a single kicker. However, the database 108 may store kick data for multiple players. For example, every player on a team may have his or her kick data stored, and the team may identify and select the preferred kicker based on the comparative kick data at the location of a current free kick.

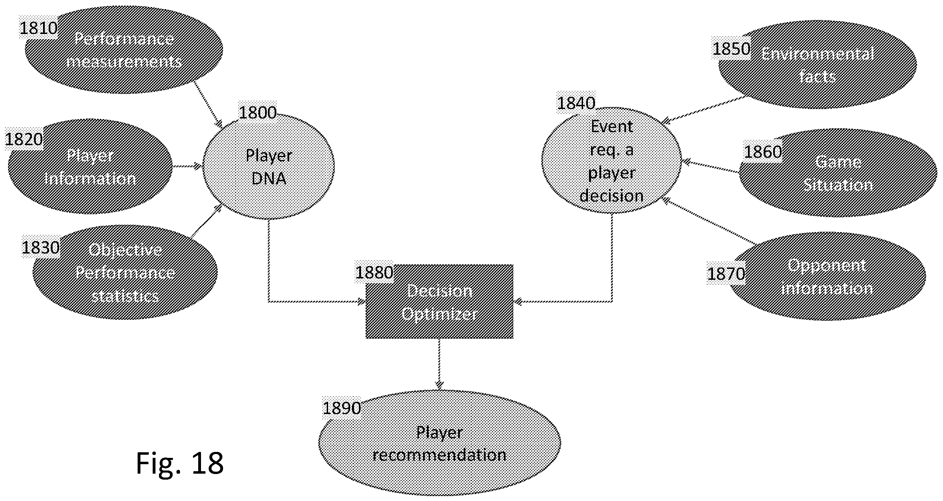

Each kicker's data may not only describe the kicking abilities of the player but may include more information than just the results of the performance measurements 1810 (that is the mentioned kick data and kick performances). For example, the data may include additional player information 1820 about the player's health or body metrics, and it could include his or her historical performance statistics 1830 from other match situations. Such player data would be useful in a number of situations e.g. from a coaching perspective, from a player development perspective, or when different players kicking abilities are being compared. In a game situation, there may be a specific event 1840 at hand which requires an evaluation of the performances of the available kickers. This event may be described not only by the kick situation, which is considered part of the game situation 1860 that may further contain information about the time left, the type of event, the score, the characteristics of the ball etc. The event 1840 may also contain information about the environment 1850 (geographical location, weather) or opponent information 1870 (specific goalkeeper information, player positions etc.). Combining the player data with the event at hand may then lead to a recommended evaluation via a decision optimizer 1880 to obtain some recommendation for the given player for the specific event at hand. Across players, this could also lead to a specific player recommendation 1890, e.g. who would be the ideal player to kick from the given free kick location given all the factors at hand, and what should be the kicking technique and speed to be applied. This generalized eco-system of such a decision evaluation is depicted in Fig. 18.

Other Applications

The principles described above are well-suited for offensive purposes, however, the principles may also be applied to defensive situations. For example, a defending team that gathers kick data on its opponents may formulate a defensive plan for defending against a direct free kick. In such a situation, a defensive team having detailed knowledge on the kicking techniques of its opponent players could configure a wall in a different manner or place a goalkeeper at a different location to optimize its chances for preventing a goal off of a direct free kick or otherwise.

The principles discussed above may be generalized to other sports as well, where some kind of a resulting ball trajectory is measured and/or evaluated based on some measured launch parameters. Examples of such sports are golf, tennis, baseball, cricket, American football, rugby, badminton, table tennis, European handball, volleyball, basketball, field hockey, where a given set of data leading to a trajectory could be evaluated to determine its performance in a given situation. That data could be optimized via an optimizer that would help the player improve his/her performance to ultimately improve the potential outcome of the given situation. In general, the invention is especially well-suited for situations where the player has time to analyze the situation and execute accordingly. In soccer that would typically be the set pieces situations like free kicks, penalty kicks, throw-ins, goalkeeper distribution, corner kicks etc. In other sports that could be serves in tennis, table tennis, badminton and volleyball, field goals, punts and kick-offs in American Football, throws in the Olympic Throwing sports. Practice situations do also in general allow players to spend time on analyzing the situation in between the action and then repeating the performance again and again, meaning that although the situation itself may not leave much time to analyze, the principles can still be applied. That applies for situations such as open play shooting, long passes, long throws, goalkeeper distribution, corner kicks, etc.

In one example, the exemplary principles may be applied to tennis. A tennis ball, like a soccer ball, has launch parameters including a vertical launch angle, a horizontal launch direction, a launch speed, a spin rate and a spin axis. A sensor may be implemented in a manner similar to the tracking system 100, and a tennis player may have swing data captured. The system may be tailored toward an optimization of, e.g., serve parameters for locating the ball in the appropriate portion of the tennis court (i.e., the target serve court) while avoiding the net and any portion covered by a team mate in doubles. In addition, sub-portions of the proper service court may be designated as less desirable as, for example, easily returnable areas. In a manner similar to that described for the soccer goalie above, the areas designated less desirable may be identified based on, historical data for an opponent, a current position of the opponent, the status of first vs. second serve, speed and skill level for the opponent, etc. The system may optimize the launch parameters by adjusting one or more of the parameters to maximize an area of the court where, if the player lands a serve within that area, the opponent would not be expected to successfully or effectively return the serve. In another embodiment, the processor may determine grades of areas for delivering the ball; for example, an area where the opponent would be expected to be able to return the ball well, return the ball adequately, return the ball poorly, or not return the ball at all. The system may further account for pre-launch parameters of the tennis ball, i.e., the toss for a serve, the speed, direction, spin, etc. of an incoming ball struck by the opposing player, etc., in estimating an area of the court that is hittable by the player.

Such a system for tennis may also have a learning feature, similar to that discussed above. A tennis player may seek to adjust his or her swing to, e.g., generate more top spin for a serve, more back/side spin for a drop shot, etc. By graphically showing the player the advantages of mastering a different type of shot the player may adjust his or her game accordingly.

Finally, the invention is also very suited for processes involving equipment optimization. This may be from the equipment maker perspective, where materials / shapes etc. can be tested and evaluated, but it can also be in fitting processes, where sports equipment is customized to match the performance of the player e.g. in golf the club's shaft length / stiffness / the club head weight / dimension / weight distribution / shape etc., in soccer the boot weight / shape / dimension / material, in racket sports the string pattern / tension / material, the racket dimensions etc.

Claims

1. A method, comprising:
   at a processor:

   determining, by a tracking device, trajectory data for a kicked ball, the trajectory data including a number of measurement times each with a corresponding three-dimensional position of the ball;
   identifying, from the trajectory data, a plurality of kick parameters for the kicked ball including a plurality of launch parameters:

   generating a series of ball trajectory simulations using the identified kick parameters in combination with an aerodynamic model wherein, for each of the simulated trajectories, a value of a first one of the identified launch parameters is adjusted to a first new value different from each of a plurality of previous values used for others of the simulated trajectories;
   determining, for each of the simulated trajectories, a simulated position of the ball relative to a target area;
   calculating, for each of the simulated trajectories, a blocked portion of the target area based on an obstacle between a launch position and the target area; and
   determining an optimized value of the first kick parameter where the simulated trajectory calculated with the optimized value enters an unblocked portion of the target area, the unblocked portion of the target area being the area remaining when the blocked portion of the target area is subtracted from the target area.

2. The method of claim 1, wherein a size of the unblocked portion of the target area based on a first simulated trajectory is different than a size of the unblocked portion of the target area based on a second simulated trajectory, wherein the optimized value of the first kick parameter is the value resulting in a greater size of the unblocked portion of the target area.

3. The method of claim 1, further comprising:

generating a further series of ball trajectory simulations using the identified kick parameters in combination with the aerodynamic model wherein, for each of the further simulated trajectories, a value of a second one of the identified launch parameters is adjusted to a second new value different from each of the first new values; determining, for each of the further simulated trajectories, a simulated position of the ball relative to the target area; calculating, for each of the further simulated trajectories, a blocked portion of the target area based on the obstacle; and determining optimized values of the first and second parameters as values of the first and second parameters resulting in a greater size of the unblocked portion of the target area.

4. The method of claim 1, wherein the launch parameters comprise an impact speed, a vertical launch angle, a horizontal launch direction, a spin rate at launch and a spin axis at launch.

5. The method of claim 4, wherein the first kick parameter is one of the vertical launch angle and the horizontal launch direction.

6. The method of claim 4, further comprising:
adjusting the impact speed and one of the spin rate at launch and the spin axis at launch to increase a size of the unblocked portion of the target area.

7. The method of claim 1, wherein the target area is a simulated goal, the obstacle is one of a simulated defensive wall and a simulated goalkeeper and the kicked ball is a football.

8. The method of claim 7, further comprising:
determining a goalkeeper area where the simulated goalkeeper is predicted to prevent a simulated trajectory of the kicked ball from entering the target area based on a time of flight for the simulated trajectory, wherein the goalkeeper area is at least part of the blocked portion of the target area.

9. The method of claim 8, wherein the goalkeeper area is further based on an initial position of the goalkeeper at a time of launch and an expected reach of the goalkeeper within the time of flight.

10. The method of claim 8, further comprising:
determining a wall area where the simulated wall is predicted to block a simulated trajectory of the kicked ball from entering the target area, the blocked portion of the target area further comprising the wall area and the unblocked portion of the target area being a goal area where the simulated goalkeeper and the simulated wall are not expected to block a simulated trajectory.

11. The method of claim 7, further comprising:

storing, on a database, a first plurality of kick parameters for a first kicker and a second plurality of kick parameters for a second kicker; and
determining which one of the first and second kickers has a greater likelihood of scoring a goal based on the launch position, a position of the obstacle and the first and second pluralities of kick parameters.

12. The method of claim 7, further comprising:

receiving location data of an actual goalkeeper and an actual defensive wall in a football match from a player tracking system; and
simulating a location of the simulated goalkeeper and the simulated defensive wall based on the location data.

13. A device, comprising:

a tracking device determining trajectory data for a kicked ball, the trajectory data including a number of measurement times each with a corresponding three-dimensional position of the ball; and
a processor identifying, from the trajectory data, a plurality of kick parameters for the kicked ball including a plurality of launch parameters, the processor generating a series of ball trajectory simulations using the identified kick parameters in combination with an aerodynamic model wherein, for each of the simulated trajectories, a value of a first one of the identified launch parameters is adjusted to a first new value different from each of a plurality of previous values used for others of the simulated trajectories, the processor determining, for each of the simulated trajectories, a simulated position of the ball relative to a target area; the processor calculating, for each of the simulated trajectories, a blocked portion of the target area based on an obstacle between a launch position and the target area; and the processor determining an optimized value of the first kick parameter where the simulated trajectory calculated with the optimized value enters an unblocked portion of the target area, the unblocked portion of the target area being the area remaining when the blocked portion of the target area is subtracted from the target area.

**Patentansprüche**

1. Verfahren, umfassend:
   bei einem Prozessor:

   Bestimmen, mittels einer Trackingvorrichtung, von Trajektoriendaten für einen geschossenen Ball, wobei die Trajektoriendaten eine Zahl von Messzeiten enthalten, jeweils mit einer entsprechenden dreidimensionalen Position des Balls;
   Identifizieren, aus den Trajektoriendaten, einer Mehrzahl von Schussparametern für den geschossenen Ball einschließlich einer Mehrzahl von Abschussparametern;
   Generieren einer Serie von Balltrajektoriensimulationen unter Verwendung der identifizierten Schussparameter in Kombination mit einem aerodynamischen Modell, wobei für jede der simulierten Trajektorien ein Wert eines ersten von den identifizierten Abschussparametern auf einen ersten neuen Wert angepasst wird, der von jedem von einer Mehrzahl von vorherigen Werten verschieden ist, die für andere von den simulierten Trajektorien verwendet werden;
   Bestimmen, für jede der simulierten Trajektorien, einer simulierten Position des Balls relativ zu einem Zielgebiet;
   Berechnen, für jede der simulierten Trajektorien, eines blockierten Bereichs des Zielgebiets basierend auf einem Hindernis zwischen einer Abschussposition und dem Zielgebiet; und
   Bestimmen eines optimierten Werts des ersten Schussparameters, wo die mit dem optimierten Wert berechnete simulierte Trajektorie in einen unblockierten Bereich des Zielgebiets eintritt, wobei der unblockierte Bereich des Zielgebiets das Gebiet ist, das verbleibt, wenn der blockierte Bereich des Zielgebiets von dem Zielgebiet subtrahiert wird.

2. Verfahren nach Anspruch 1, wobei eine Größe des unblockierten Bereichs des Zielgebiets basierend auf einer ersten simulierten Trajektorie verschieden ist von einer Größe des unblockierten Bereichs des Zielgebiets basierend auf einer zweiten simulierten Trajektorie, wobei der optimierte Wert des ersten Schussparameters der Wert ist, der in einer größeren Größe des unblockierten Bereichs des Zielgebiets resultiert.

3. Verfahren nach Anspruch 1, ferner umfassend:

   Generieren einer weiteren Serie von Balltrajektoriensimulationen unter Verwendung der identifizierten Schussparameter in Kombination mit dem aerodynamischen Modell, wobei für jede der weiteren simulierten Trajektorien ein Wert eines zweiten von den identifizierten Abschussparametern auf einen zweiten neuen Wert angepasst wird, der von jedem der ersten neuen Werte verschieden ist;
   Bestimmen, für jede der weiteren simulierten Trajektorien, einer simulierten Position des Balls relativ zu dem Zielgebiet;
   Berechnen, für jede der weiteren simulierten Trajektorien, eines blockierten Bereichs des Zielgebiets basierend auf dem Hindernis; und
   Bestimmen von optimierten Werten der ersten und zweiten Parameter als Werte der ersten und zweiten Parameter, die in einer größeren Größe des unblockierten Bereichs des Zielgebiets resultieren.

4. Verfahren nach Anspruch 1, wobei die Abschussparameter eine Aufprallgeschwindigkeit, einen vertikalen Abschusswinkel, eine horizontale Abschussrichtung, eine Spinrate beim Abschuss und eine Spinachse beim Abschuss umfassen.

5. Verfahren nach Anspruch 4, wobei der erste Schussparameter einer von dem vertikalen Abschusswinkel und der horizontalen Abschussrichtung ist.

**6.** Verfahren nach Anspruch 4, ferner umfassend:
Anpassen der Aufprallgeschwindigkeit und eines von der Spinrate beim Abschuss und der Spinachse beim Abschuss, um eine Größe des unblockierten Bereichs des Zielgebiets zu vergrößern.

**7.** Verfahren nach Anspruch 1, wobei das Zielgebiet ein simuliertes Tor ist, das Hindernis eines von einer simulierten Abwehrmauer und einem simulierten Torhüter ist, und der geschossene Ball ein Fußball ist.

**8.** Verfahren nach Anspruch 7, ferner umfassend:
Bestimmen eines Torhütergebiets, wo vorhergesagt wird, dass der simulierte Torhüter verhindert, dass eine simulierte Trajektorie des geschossenen Balls in das Zielgebiet eintritt, basierend auf einer Flugzeit für die simulierte Trajektorie, wobei das Torhütergebiet wenigstens ein Teil des blockierten Bereichs des Zielgebiets ist.

**9.** Verfahren nach Anspruch 8, wobei das Torhütergebiet ferner auf einer anfänglichen Position des Torhüters zu einem Abschusszeitpunkt und einer erwarteten Reichweite des Torhüters innerhalb der Flugzeit basiert.

**10.** Verfahren nach Anspruch 8, ferner umfassend:
Bestimmen eines Mauergebiets, wo vorhergesagt wird, dass die simulierte Mauer eine simulierte Trajektorie des geschossenen Balls vom Eintreten in das Zielgebiet blockiert, wobei der blockierte Bereich des Zielgebiets ferner das Mauergebiet umfasst, und der unblockierte Bereich des Zielgebiets ein Torgebiet ist, wo nicht erwartet wird, dass der simulierte Torhüter und die simulierte Mauer eine simulierte Trajektorie blockieren.

**11.** Verfahren nach Anspruch 7, ferner umfassend:

Speichern, in einer Datenbank, einer ersten Mehrzahl von Schussparametern für einen ersten Schützen, und einer zweiten Mehrzahl von Schussparametern für einen zweiten Schützen; und
Bestimmen, welcher von dem ersten und dem zweiten Schützen eine größere Wahrscheinlichkeit für die Erzielung eines Tors hat, basierend auf der Abschussposition, einer Position des Hindernisses und der ersten und der zweiten Mehrzahl von Schussparametern.

**12.** Verfahren nach Anspruch 7, ferner umfassend:

Empfangen von Ortsdaten eines tatsächlichen Torhüters und einer tatsächlichen Abwehrmauer in einem Fußballspiel von einem Spielertrackingsystem; und
Simulieren eines Orts des simulierten Torhüters und der simulierten Abwehrmauer basierend auf den Ortsdaten.

**13.** Vorrichtung, umfassend:

eine Trackingvorrichtung, die Trajektoriendaten für einen geschossenen Ball bestimmt, wobei die Trajektoriendaten eine Zahl von Messzeiten enthalten, jeweils mit einer entsprechenden dreidimensionalen Position des Balls; und
einen Prozessor, der aus den Trajektoriendaten eine Mehrzahl von Schussparametern für den geschossenen Ball einschließlich einer Mehrzahl von Abschussparametern identifiziert, wobei der Prozessor eine Serie von Balltrajektoriensimulationen generiert unter Verwendung der identifizierten Schussparameter in Kombination mit einem aerodynamischen Modell, wobei für jede der simulierten Trajektorien ein Wert eines ersten von den identifizierten Abschussparametern auf einen ersten neuen Wert angepasst wird, der verschieden ist von jedem von einer Mehrzahl von vorherigen Werten, die für andere von den simulierten Trajektorien verwendet werden, wobei der Prozessor für jede der simulierten Trajektorien eine simulierte Position des Balls relativ zu einem Zielgebiet bestimmt; wobei der Prozessor für jede der simulierten Trajektorien einen blockierten Bereich des Zielgebiets berechnet basierend auf einem Hindernis zwischen einer Abschussposition und dem Zielgebiet; und wobei der Prozessor einen optimierten Wert des ersten Schussparameters bestimmt, wo die mit dem optimierten Wert berechnete simulierte Trajektorie in einen unblockierten Bereich des Zielgebiets eintritt, wobei der unblockierte Bereich des Zielgebiets das Gebiet ist, das verbleibt, wenn der blockierte Bereich des Zielgebiets von dem Zielgebiet subtrahiert wird.

**Revendications**

**1.** Un procédé comprenant :

au niveau d'un processeur ;

le fait, par un dispositif de suivi, de déterminer des données de trajectoire pour un ballon frappé, les données de trajectoire comprenant un certain nombre de temps de mesure, chacun avec une position tridimensionnelle correspondante du ballon ;

le fait, à partir des données de trajectoire, d'identifier une pluralité de paramètres de frappe du ballon frappé incluant une pluralité de paramètres de lancement ;

le fait de générer une série de simulations de trajectoires de balle en utilisant les paramètres de frappe identifiés en combinaison avec un modèle aérodynamique dans lequel, pour chacune des trajectoires simulées, une valeur d'un premier des paramètres de lancement identifiés est ajustée à une première nouvelle valeur différente à partir de chacune d'une pluralité de valeurs précédentes utilisées pour d'autres des trajectoires simulées ;

le fait, pour chacune des trajectoires simulées, de déterminer une position simulée du ballon par rapport à une zone cible ;

le fait, pour chacune des trajectoires simulées, de calculer une portion bloquée de la zone cible en fonction d'un obstacle entre une position de lancement et la zone cible ; et

le fait de déterminer une valeur optimisée du premier paramètre de frappe selon laquelle la trajectoire simulée calculée avec la valeur optimisée entre dans une partie non bloquée de la zone cible, la partie non bloquée de la zone cible étant la zone restant lorsque la partie bloquée de la zone cible est soustraite de la zone cible.

2. Le procédé selon la revendication 1, dans lequel une taille de la partie non bloquée de la zone cible basée sur une première trajectoire simulée est différente d'une taille de la partie non bloquée de la zone cible basée sur une deuxième trajectoire simulée, la valeur optimisée du premier paramètre de frappe étant la valeur résultant en une plus grande taille de la partie non bloquée de la zone cible.

3. Le procédé selon la revendication 1, comprenant en outre :

le fait de générer une autre série de simulations de trajectoire de balle en utilisant les paramètres de frappe identifiés en combinaison avec le modèle aérodynamique, dans lequel, pour chacune des autres trajectoires simulées, une valeur d'un deuxième des paramètres de lancement identifiés est ajustée à un deuxième nouvelle valeur différente de chacune des premières nouvelles valeurs ;

le fait, pour chacune des autres trajectoires simulées, de déterminer une position simulée du ballon par rapport à la zone cible ;

le fait, pour chacune des autres trajectoires simulées, de calculer une portion bloquée de la zone cible en fonction de l'obstacle ; et

le fait de déterminer des valeurs optimisées des premier et deuxième paramètres en tant que valeurs des premier et deuxième paramètres entraînant une plus grande taille de la partie non bloquée de la zone cible.

4. Le procédé selon la revendication 1, dans lequel les paramètres de lancement comprennent une vitesse d'impact, un angle de lancement vertical, une direction de lancement horizontale, une vitesse de rotation au lancement et un axe de rotation au lancement.

5. Le procédé selon la revendication 4, dans lequel le premier paramètre de frappe est l'un parmi l'angle de lancement vertical et la direction de lancement horizontale.

6. Le procédé selon la revendication 4, comprenant en outre :
le fait d'ajuster la vitesse d'impact et un parmi la vitesse de rotation au lancement et l'axe de rotation au lancement pour augmenter une taille de la partie non bloquée de la zone cible.

7. Le procédé selon la revendication 1, dans lequel la zone cible est un but simulé, l'obstacle est celui d'un mur défensif simulé et d'un gardien de but simulé et le ballon frappé est un ballon de football.

8. Le procédé selon la revendication 7, comprenant en outre :
le fait de déterminer une zone de gardien de but dans laquelle il est prévu que le gardien de but simulé empêche qu'une trajectoire simulée du ballon frappé n'entre dans la zone cible sur la base d'un temps de vol pour la trajectoire simulée, la zone de gardien de but étant au moins une partie de la partie bloquée de la zone cible.

9. Le procédé selon la revendication 8, dans lequel la zone de gardien de but est en outre basée sur une position initiale du gardien de but au moment du lancement et une portée d'intervention attendue du gardien de but pendant le temps de vol.

10. Le procédé selon la revendication 8, comprenant en outre :
le fait de déterminer une zone de mur où le mur simulé est censé empêcher qu'une trajectoire simulée du ballon frappé n'entre dans la zone cible, la partie bloquée de la zone cible comprenant en outre la zone de mur et la partie non bloquée de la zone cible étant une zone de but où le gardien de but simulé et le mur simulé ne sont pas censés bloquer une trajectoire simulée.

11. Le procédé selon la revendication 7, comprenant en outre :

le fait de stocker sur une base de données une première pluralité de paramètres de frappe pour un premier tireur et une deuxième pluralité de paramètres de frappe pour un deuxième tireur ; et
le fait de déterminer lequel des premier et deuxième tireurs a la plus grande probabilité de marquer un but sur la base de la position de lancement, d'une position de l'obstacle et de la première et de la deuxième pluralité de paramètres de frappe.

12. Le procédé selon la revendication 7, comprenant en outre :

le fait de recevoir des données de localisation d'un véritable gardien de but et d'un véritable mur défensif lors d'un match de football à partir d'un système de suivi des joueurs ; et
le fait de simuler un emplacement du gardien de but simulé et du mur défensif simulé sur la base des données de localisation.

13. Un dispositif, comprenant :

un dispositif de suivi qui détermine des données de trajectoire pour un ballon frappé, les données de trajectoire comprenant un certain nombre de temps de mesure avec chacun une position tridimensionnelle correspondante du ballon ; et
un processeur identifiant, à partir des données de trajectoire, une pluralité de paramètres de frappe pour le ballon frappé comprenant une pluralité de paramètres de lancement, le processeur générant une série de simulations de trajectoire de ballon en utilisant des paramètres de frappe identifiés en combinaison avec un modèle aérodynamique ; pour chacune des trajectoires simulées, une valeur d'un premier des paramètres de frappe identifiés est ajustée à une première nouvelle valeur différente de chacune d'une pluralité de valeurs précédentes utilisées pour d'autres des trajectoires simulées, le processeur déterminant, pour chacune trajectoire parmi les trajectoires simulées, une position simulée du ballon par rapport à une zone cible ; le processeur calcule, pour chacune des trajectoires simulées, une partie bloquée de la zone cible en fonction d'un obstacle entre une position de lancement et la zone cible ; et le processeur détermine une valeur optimisée du premier paramètre de frappe selon laquelle la trajectoire simulée calculée avec la valeur optimisée entre dans une partie non bloquée de la zone cible, la partie non bloquée de la zone cible étant la zone restante lorsque la partie bloquée de la zone cible est soustraite de la zone cible.

Fig. 1

Fig. 2

Fig. 3

Fig. 4

Fig. 5

Spin Axis vs Vertical Launch Angle    Spin Rate vs Vertical Launch Angle    Ball Speed  vs Vertical Launch Angle

$y = 0.0304x + 22.212$

$y = -0.0081x + 23.637$

$y = -0.1152x + 25.596$

## Fig. 6

EP 3 768 398 B1

Fig. 7

Fig. 8

EP 3 768 398 B1

20 m

First line-of-sight

$t_{blocked}$　　$t_{reaction}$　　$t_{move}$　　$t_{jump}$　Flight time

Fig. 9

Fig. 10

Fig. 11

Fig. 12

Fig. 13

Fig. 14

Fig. 15

EP 3 768 398 B1

Fig. 16

1705 — Calculate Kick Performance before optimization

Goal Keeper Area

Goal Area

Wall Area

1715 — Is *Goal Area* Maximized?

Yes

No

1710 — Calculate adj. Kick Performance

Adj. kick data

Maximized Kick Performance

Goal Keeper Area

Goal Area

Wall Area

- Maximized Kick Data
- Maximized Kick Performance

Fig. 17

Fig. 18

**EP 3 768 398 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018085894 A1

- US 8845442 B

**Non-patent literature cited in the description**

- Measuring and Modelling the Goalkeeper's Diving Envelope in a Penalty Kick. **KERWIN D.G ; BRAY K.** The Engineering of Sport. Springer, 2006, vol. 6